# EUROPÄISCHE PATENTANMELDUNG

(11) **EP 4 043 789 A1**
(43) Veröffentlichungstag der Anmeldung: **17.08.2022**
(21) Anmeldenummer: 22151738.6
(22) Anmeldetag: 17.01.2022
(51) Int. Cl.: F23D 14/02, F23L 5/02, F23N 5/00, F23N 5/24, G01N 33/00, G01N 27/04, G01N 27/12

(54) **VERFAHREN UND ANORDNUNG ZUM ERKENNEN UND/ODER BEOBACHTEN VON FLAMMEN IN EINEM HEIZGERÄT**

(30) Priorität: 22.01.2021 DE 102021101360
(71) Anmelder: Vaillant GmbH, 42859 Remscheid (DE)
(72) Erfinder: Reinert, Andreas, 58455 Witten (DE); Wohlfeil, Arnold, 42799 Leichlingen (DE); Richter, Klaus, 42855 Remscheid (DE); Grabe, Jochen, 51688 Wipperfürth (DE); Hanfland, Sven, 42653 Solingen (DE); Oerder, Bodo, 42897 Remscheid (DE); Tomczak, Heinz-Jörg, 42327 Wuppertal (DE); Altendorf, Frank, 51467 Bergisch Gladbach (DE); Salg, Frank, 42897 Remscheid (DE); Paul, Michael, 58332 Schwelm (DE)
(74) Vertreter: Popp, Carsten

(57) **Zusammenfassung**

Die Erfindung betrifft ein Verfahren und eine Anordnung zum Erkennen und/oder Überwachen von Flammen (9) in einem Verbrennungsraum (8) eines mit Wasserstoff betreibbaren Heizgerätes (1), wobei dem Verbrennungsraum (8) zugeführtes Gemisch aus Luft und Wasserstoff in dem Verbrennungsraum (8) unter Bildung von Flammen (9) verbrannt wird und entstehende Verbrennungsgase durch eine Abgasanlage (10) abgeführt werden und wobei in der Abgasanlage (10) mindestes ein für Wasserstoff empfindlicher elektrochemischer Sensor (11), nämlich ein Halbleitersensor auf Basis von Galliumoxid, angeordnet wird, der von den Verbrennungsgasen umströmt wird und dessen Messignal zum Erkennen des Vorhandenseins von Flammen (9) und/oder der Qualität der Verbrennung ausgewertet wird. Die vorliegende Erfindung erlaubt es, mit einer einfachen und robusten Instrumentierung an einem Heizgerät (1) eine Flammenüberwachung durchzuführen, was für Wasserstoff als Brenngas von Vorteil ist, wo andere bekannte Messverfahren nicht eingesetzt werden können.

## Beschreibung

Die Erfindung betrifft ein Verfahren und eine Anordnung zum Erkennen und/oder zum Beobachten von Flammen in einem Heizgerät, nämlich einem, das mit (reinem) Wasserstoff als Brenngas betreibbar ist. Wasserstoff als Brenngas wird immer wichtiger, und es werden große Anstrengungen unternommen, neue oder auch existierende Heizgeräte für einen Betrieb damit zu ertüchtigen. Dabei geht es nicht nur um große Anlagen, sondern auch um Wandgeräte zur Erwärmung von Wasser und generell um Heizgeräte für die Beheizung von Gebäuden und/oder die Bereitstellung von warmem Wasser.

Wasserstoff unterscheidet sich bei seiner Verbrennung in mehreren Punkten von bisher verwendeten Brenngasen, insbesondere ist eine Wasserstofflamme für das menschliche Auge fast unsichtbar, strahlt weniger Wärme ab als mit kohlenstoffhaltigen Brennstoffen erzeugte Flammen, und es werden andere Messsysteme benötigt als bei Heizgeräten für Brennstoffe aus Kohlenwasserstoffen. Die vorliegende Erfindung ist daher besonders geeignet für Heizgeräte, die mit reinem Wasserstoff betrieben werden.

In Heizgeräten werden bisher im Allgemeinen einfache und robuste Sensoren für Temperatur, Licht- oder Wärme-Strahlung, Druck, Volumenstrom und dergleichen eingesetzt, um die Heizgeräte zu regeln und deren sicheren Betrieb zu gewährleisten. Mit bisher üblicher Sensorik lassen sich jedoch bei Verwendung von Wasserstoff als Brenngas manche Messungen nicht zuverlässig durchführen. Eine wichtige Aufgabe ist das Feststellen des Vorhandenseins einer stabilen Flamme (ein sogenannter Flammenwächter), eine andere die Einstellung eines für eine stabile und umweltschonende Verbrennung geeigneten Verhältnisses von Verbrennungsluft zu Brenngas (Lambda-Wert).

Bei Heizgeräten nach dem Stand der Technik werden für die Überwachung der Verbrennung oft lonisationsmessgeräte und/oder optische Sensoren eingesetzt, mit denen sich bei kohlenwasserstoffhaltigen Brenngasen das Vorhandensein einer Flamme und/oder bestimmte Parameter bei einer Verbrennung detektieren lassen. Je mehr Wasserstoff in einem Brenngas vorhanden ist, desto weniger gut lassen sich diese Messmethoden jedoch anwenden. In der älteren, nicht vorveröffentlichten Patentanmeldung DE 102020129162 A1 ist bereits die Messung von Wasserstoff im Abgas eines Heizgerätes mittels optischer Verfahren beschrieben, wobei diese Messung auch als Ersatz für bisherige Flammenwächter dient.

Aus verschiedenen technischen Gebieten sind auch elektrochemische Sensoren bekannt, die für Wasserstoff empfindlich sind. Solche Sensoren sind beispielsweise in der DE 10 105 581 C1 beschrieben.

Aufgabe der vorliegenden Erfindung ist es, die mit Bezug auf den Stand der Technik geschildeten Probleme wenigstens teilweise zu lösen, und insbesondere die Schaffung eines Verfahrens und einer Anordnung zum Feststellen des Vorhandenseins und/oder zum Beobachten von Flammen in einem Verbrennungsraum eines Heizgerätes auf nicht optischer Basis, wobei die Anordnung einfach und geeignet für einen Alltagsbetrieb eines Heizgerätes sein soll.

Zur Lösung dieser Aufgabe dienen ein Verfahren und eine Anordnung sowie ein Computerprogrammprodukt gemäß den unabhängigen Ansprüchen. Vorteilhafte Ausgestaltungen und Weiterbildungen der Erfindung sind in den jeweiligen abhängigen Ansprüchen angegeben. Die Beschreibung, insbesondere im Zusammenhang mit der Zeichnung, veranschaulicht die Erfindung und gibt weitere Ausführungsbeispiele an.

Bei dem vorgeschlagenen Verfahren zum Erkennen und/oder Überwachen von Flammen in einem Verbrennungsraum eines mit (im Wesentlichen reinen) Wasserstoff betreibbaren Heizgerätes, wird dem Verbrennungsraum zugeführtes Gemisch aus Luft und Brenngas in dem Verbrennungsraum unter Bildung von Flammen verbrannt, und entstehende Verbrennungsgase werden durch eine Abgasanlage abgeführt, wobei in der Abgasanlage mindestes ein für Wasserstoff empfindlicher elektrochemischer Sensor, nämlich ein Halbleitersensor auf Basis von Galliumoxid angeordnet wird, der von den Verbrennungsgasen umströmt wird und dessen Messignal zum Erkennen des Vorhandenseins von Flammen und/oder der Qualität der Verbrennung ausgewertet wird. Solche elektrochemische Sensoren sind robust und können z. B. über zweiadrige Messleitungen mit einer Steuer- und Regeleinheit verbunden werden. Typischerweise ändert sich ihr elektrischer Widerstand in Abhängigkeit von den chemischen und/oder physikalischen Eigenschaften der sie umgebenden Gase, was im vorliegenden Fall das zuverlässige Erkennen von Wasserstoff erlaubt und in gewissen Grenzen auch eine quantitative Messung einer Wasserstoffkonzentration im Abgas.

Als ein für Wasserstoff empfindlicher Sensor wird ein Halbleitersensor verwendet, dessen Widerstand sich mit der Konzentration von reduzierendem Gas in seiner Umgebung ändert. Es braucht daher nur der Widerstand des Sensors beobachtet zu werden, um daraus auf die Konzentration von Wasserstoff (das ist bei mit Wasserstoff betriebenen Heizgeräten praktisch das einzige reduzierende Gas, welches in der Abgasanlage auftreten kann) im Abgas zu schließen.

Besonders geeignet für solche Messungen sind Halbleitersensoren, die auf der Basis von Galliumoxid hergestellt sind. Bei diesen ändert sich der elektrische Widerstand stark und reproduzierbar mit der Konzentration von Wasserstoff in der Umgebung, mit der sie in Kontakt sind.

Besonders vorteilhaft ist es, während eines Zündvorganges der Verbrennung das Messsignal und/oder die zeitliche Veränderung des Messsignals des für Wasserstoff empfindlichen Sensors zu beobachten, wobei bei einer Änderung oberhalb eines Schwellwertes auf das Ausbleiben einer Zündung des Gemisches geschlossen wird. Sobald die Zufuhr von Gemisch mit Anteilen von Wasserstoff zum Brennraum gestartet wird, kann dieses mit einer bauartbedingten Verzögerung in die Abgasanlage gelangen, sofern es nicht vorher erfolgreich gezündet und verbrannt wird. Ein Anstieg erfolgt also, wenn eine Zündung nicht frühzeitig erfolgt. Falls also nach Starten der Zufuhr von Gemisch ein Anstieg an Wasserstoff gemessen wird und dieser nicht innerhalb eines vorgebbaren Sicherheitsintervalls wieder abfällt, kann daraus auf das Ausbleiben einer Zündung geschlossen werden. Aus der Dauer eines gemessenen Anstiegs an Wasserstoff kann auch auf die Qualität des Zündvorganges geschlossen werden. Die zeitliche Ableitung des gemessenen Widerstandes erlaubt eine besonders empfindliche Messung.

Außerdem kann bevorzugt während des Vorhandenseins von Flammen, also bei laufender Verbrennung im Verbrennungsraum, die das Messsignal und/oder die zeitliche Veränderung des Messsignals des für Wasserstoff empfindlichen Sensors beobachtet werden, wobei bei einer zeitlichen Änderung oberhalb eines Schwellwertes auf ein Erlöschen der Flammen geschlossen wird. Falls bei weiter zugeführtem Gemisch mit Anteilen an Wasserstoff die Flammen im Verbrennungsraum erlöschen, gelangt sehr schnell unverbrannter Wasserstoff in die Abgasanlage, wo er detektiert wird und z. B. ein sofortiges Schließen eines Brenngasventils auslösen kann. Der Sensor kann also die Funktion eines Flammenwächters bei einem Zündvorgang und beim Betrieb erfüllen, also das Auftreten von Flammen und deren unerwünschtes Erlöschen detektieren, was für den sicheren Betrieb eines Heizgerätes erforderlich ist.

Es sei darauf hingewiesen, dass auch bei laufender Verbrennung nicht immer aller Wasserstoff aus einem zugeführten Gemisch verbrannt wird, obwohl eine vollständige Verbrennung das Ziel bei der Regelung der Verbrennung ist. Auch für solche Fälle kann der Sensor wichtige Funktionen im Rahmen seiner Messgenauigkeit übernehmen. Er kann zwar im Allgemeinen nicht allein für die Regelung der Gemischbildung herangezogen werden, weil er eher zu geringe als zu hohe Anteile an Verbrennungsluft im Gemisch detektieren kann, aber mit seiner Hilfe kann zumindest festgestellt werden, ob eine vollständige Verbrennung abläuft. Je nach seiner Empfindlichkeit kann der Sensor auch bei ausgeschaltetem Heizgerät Leckagen, die zum Eindringen von Wasserstoff in den Verbrennungsraum und damit in die Abgasanlage führen, erkennen und z. B. eine Warnmeldung auslösen.

Bei einer hier vorgeschlagenen Anordnung zum Erkennen und/oder Überwachen von Flammen in einem Verbrennungsraum eines Heizgerätes mit einer Abgasanlage, ist in der Abgasanlage mindestens ein für Wasserstoff empfindlicher elektrochemischer Sensor, nämlich ein Halbleitersensor auf Basis von Galliumoxid angeordnet und mit einer Steuer- und Regeleinheit verbunden, die eingerichtet ist, die Messwerte des Sensors und/oder deren zeitliches Verhalten auszuwerten und für die Überwachung oder/oder Steuerung und/oder Regelung des Heizgerätes zu verwenden.

Hier ist der elektrochemische Sensor ein Halbleitersensor, und er enthält Galliumoxid. Solche Sensoren haben einen von der Konzentration reduzierender Gase in ihrer Umgebung abhängigen Widerstand, der gemessen werden kann.

In einer typischen Ausführungsform ist der für Wasserstoff empfindliche Sensor möglichst nahe am Verbrennungsraum angeordnet ist, jedoch in einem Bereich mit für den Sensor zulässigen Temperaturbedingungen. Je näher am Verbrennungsraum der Sensor liegt, desto schneller reagiert er auf Änderungen der Wasserstoffkonzentration, die vom Verbrennungsraum ausgehen. Andererseits sind Halbleiter temperaturempfindlich, so dass bei ihrer Positionierung beachtet werden muss, dass beim Betrieb der Heizungsanlage unter keinen Bedingungen die maximal für den Sensor zulässigen Temperaturen überschritten werden.

In einer besonderen Ausführungsform können aus Gründen der Redundanz und/oder zur Erhöhung der Messgenauigkeit und/oder Aussagekraft der Messungen zwei oder mehr Sensoren vorhanden und mit der Steuer- und Regeleinheit verbunden sein, deren Messwerte alternativ oder additiv auswertbar sind. Das können gleichartige oder auch diversitär zueinander ausgelegte Sensoren sein.

Ein weiterer Aspekt betrifft ein Computerprogramprodukt umfassend Befehle, die bewirken, dass die beschriebene Anordnung das beschriebene Verfahren ausführt. Die Auswertung der gemessenen Daten und deren weitere Verwendung im Heizgerät benötigen ein Programm und Daten für die Steuerung des Heizgerätes, wobei beides gelegentlich aktualisiert werden muss.

Die Erläuterungen zum Verfahren können zur näheren Charakterisierung der Anordnung herangezogen werden, und umgekehrt. Die Anordnung kann auch so eingerichtet sein, dass damit das Verfahren durchgeführt wird.

Ein schematisches Ausführungsbeispiel der Erfindung, auf das diese jedoch nicht beschränkt ist, und die Funktionsweise des Verfahrens werden nun anhand der Zeichnung näher erläutert. Es stellt dar:

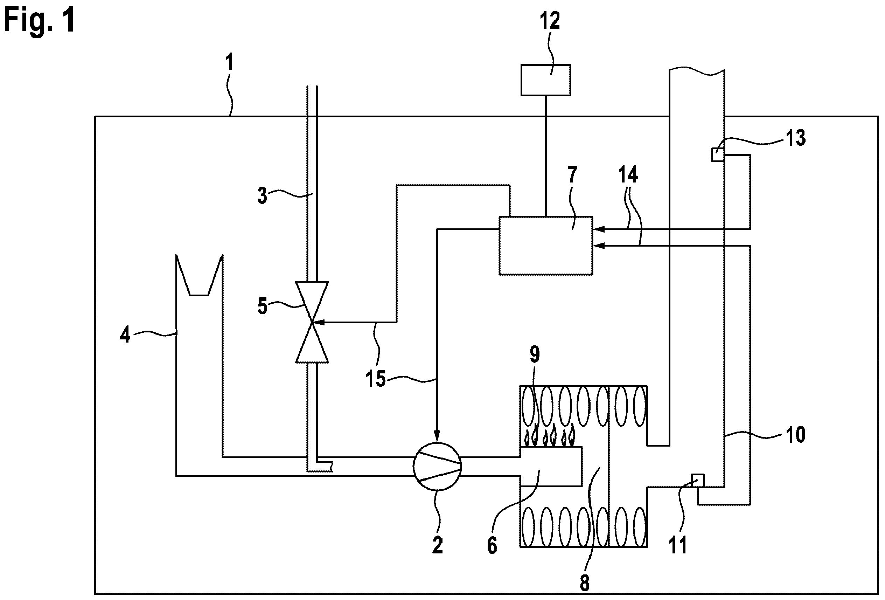

- Fig. 1:: ein Heizgerät mit einem Messsystem.

Fig. 1 zeigt schematisch ein Heizgerät 1, insbesondere eines, welches mit Wasserstoff oder einem wasserstoffhaltigen Brenngas betreibbar ist. Über eine Luftzufuhr 4 wird Luft (meist Außenluft / Umgebungsluft) von einem Gebläse 2 angesaugt und in einen Brenner 6 gefördert. Über eine Brenngasversorgung 3 und ein Brenngasventil 5 wird dem vom Gebläse 2 erzeugten Luftstrom Brenngas beigemischt. Das Gemisch tritt aus dem Brenner 6 in einen Verbrennungsraum 8 aus und wird dort verbrannt, wobei Flammen 9 auftreten. Entstehende Verbrennungsgase werden über eine Abgasanlage 10 abgeführt. In der Abgasanlage 10 ist möglichst nahe an dem Verbrennungsraum 8 ein für Wasserstoff empfindlicher elektrochemischer Sensor 11 angeordnet, nämlich ein Galliumoxid enthaltender Halbleitersensor. Dieser ändert seinen elektrischen Widerstand in Abhängigkeit von der Konzentration reduzierender Bestandteile in der Umgebung, mit der er in Kontakt steht. Hier reagiert der Sensor 11 im Wesentlichen auf die Konzentration von Wasserstoff im Abgas, da andere reduzierende Gase bei Verwendung von reinem Wasserstoff als Brenngas (in Mischung mit Luft) nicht auftreten können. Dies erlaubt seinen Einsatz als sogenannter Flammenwächter, da er detektieren kann, wenn bei einem Zündvorgang Wasserstoff (zu lange) in die Abgasanlage strömt, was als Fehlen von Flammen 9 im Verbrennungsraum interpretierbar ist. Er kann auch das ungewollte Erlöschen der Flammen 9 beim Betrieb detektieren, da dann Wasserstoff in die Abgasanlage gelangt. Eine Steuer- und Regeleinheit 7 ist über eine Messleitung 14 mit dem Sensor 11 verbunden und wertet die Messsignale des Sensors 11 aus. Sie kann daraus z. B. Befehle ableiten, die über Steuerleitungen 15 an das Gebläse 2 und/oder das Brenngasventil 5 geleitet werden. Zusätzlich kann eine Warn- oder Störmeldung an eine Anzeige 12 weitergegeben werden. Je nach Anforderungen an Messgenauigkeit und Redundanz kann mindestens ein zusätzlicher (gleichartiger oder anderer) Sensor 13 vorhanden sein, der im vorliegenden Ausführungsbeispiel an einer Stelle der Abgasanlage 10 mit niedrigerer Temperatur, dafür aber auch längerer Ansprechzeit angeordnet und ebenfalls über eine Messleitung 14 mit der Steuer- und Regeleinheit 7 verbunden ist.

Die vorliegende Erfindung erlaubt es, mit einer einfachen und robusten Instrumentierung an einem Heizgerät eine Flammenüberwachung durchzuführen, was bei Wasserstoff als Brenngas von Vorteil ist, wo andere bekannte Messverfahren nicht eingesetzt werden können.

### Bezugszeichenliste

- 1: Heizgerät
- 2: Gebläse
- 3: Brenngasversorgung
- 4: Luftzufuhr
- 5: Brenngasventil
- 6: Brenner
- 7: Steuer- und Regeleinheit
- 8: Verbrennungsraum
- 9: Flammen
- 10: Abgasanlage
- 11: Elektrochemischer Sensor
- 12: Anzeige
- 13: Zusätzlicher Sensor
- 14: Messleitungen
- 15: Steuerleitungen

## Patentansprüche

1. Verfahren zum Erkennen und/oder Überwachen von Flammen (9) in einem Verbrennungsraum (8) eines mit Wasserstoff betreibbaren Heizgerätes (1), wobei dem Verbrennungsraum (8) zugeführtes Gemisch aus Luft und Brenngas in dem Verbrennungsraum (8) unter Bildung von Flammen (9) verbrannt wird und entstehende Verbrennungsgase durch eine Abgasanlage (10) abgeführt werden, wobei in der Abgasanlage (10) mindestes ein für Wasserstoff empfindlicher elektrochemischer Sensor (11) angeordnet wird, der von den Verbrennungsgasen umströmt wird und dessen Messignal zum Erkennen des Vorhandenseins von Flammen (9) und/oder der Qualität der Verbrennung ausgewertet wird und wobei als für Wasserstoff empfindliche Sensor (11) ein Halbleitersensor auf der Basis von Galliumoxid verwendet wird, dessen elektrischer Widerstand sich mit der Konzentration von reduzierendem Gas in seiner Umgebung ändert.

2. Verfahren nach Anspruch 1, wobei während eines Zündvorganges der Verbrennung das Messsignal und/oder die zeitliche Veränderung des Messsignals des für Wasserstoff empfindlichen Sensors (11) beobachtet wird und bei einer Änderung oberhalb eines Schwellwertes auf das Ausbleiben einer Zündung des Gemisches geschlossen wird.

3. Verfahren nach einem der vorhergehenden Ansprüche, wobei während des Vorhandenseins von Flammen (9) das Messsignal und/oder die zeitliche Veränderung des Messsignals des für Wasserstoff empfindlichen Sensors (11) beobachtet wird und bei einer zeitlichen Änderung oberhalb eines Schwellwertes auf ein Erlöschen der Flammen (9) geschlossen wird.

4. Anordnung zum Erkennen und/oder Überwachen von Flammen (9) in einem Verbrennungsraum (8) eines Heizgerätes (1) mit einer Abgasanlage (10), wobei in der Abgasanlage (10) mindestens ein für Wasserstoff empfindlicher elektrochemischer Sensor (11), nämlich ein Halbleitersensor auf der Basis von Galliumoxid angeordnet und mit einer Steuer- und Regeleinheit (7) verbunden ist, die eingerichtet ist die Messwerte des Sensors (11) und oder deren zeitliches Verhalten auszuwerten und für die Überwachung und/oder Steuerung und/oder Regelung des Heizgerätes zu verwenden.

5. Anordnung nach Anspruch 4, wobei der für Wasserstoff empfindliche Sensor (11) möglichst nahe am Verbrennungsraum (8) angeordnet ist, jedoch in einem Bereich mit für den Sensor (11) zulässigen Temperaturbedingungen.

6. Anordnung nach einem der Ansprüche 4 oder 5, wobei ein zusätzlicher mit der Steuer- und Regeleinheit (7) verbundener Sensor (13) vorhanden ist, dessen Messwerte alternativ oder additiv auswertbar sind.

7. Computerprogramprodukt umfassend Befehle, die bewirken, dass die Anordnung nach einem der Ansprüche 4 bis 6 das Verfahren nach einem der Ansprüche 1 bis 3 ausführt.
